# EUROPEAN PATENT APPLICATION

(11) **EP 2 253 700 A1**
(43) Date of publication of application: **24.11.2010**
(21) Application number: 09006496.5
(22) Date of filing: 13.05.2009
(51) Int. Cl.: C12N 5/071

(54) **A method for producing test systems from donors suffering from adverse effects of medicaments and /or medical treatments, and uses of said systems**

(71) Applicant: Helmholtz-Zentrum für Infektionsforschung GmbH, 38124 Braunschweig (DE); Medizinische Hochschule Hannover, 30625 Hannover (DE)
(72) Inventor: May, Tobias, 38124 Braunschweig (DE); Hauser, Hansjörg, 38302 Wolfenbüttel (DE); Wirth, Dagmar, 38124 Braunschweig (DE); Cantz, Tobias, 30625 Hannover (DE)
(74) Representative: Krauss, Jan

(57) **Abstract**

The present invention relates to an *in vitro* method for producing induced stem cells (iSCs), in particular induced pluripotent stem (iPS) cells, derived from an mammal which suffers from at least one adverse effect caused by a medical treatment of a disease in said mammal, wherein said mammal has a genetic defect or genetic predisposition that is linked with said adverse effect against said medical treatment, comprising reprogramming of somatic cells obtained from said mammal with at least one suitable reprogramming factor. The present invention further relates to iSC-cell lines, preferably iPS-cell lines and their uses in screening methods in the context of searching for treatments lacking or having reduced side-effects.

## Description

The present invention relates to an *in vitro* method for producing induced stem cells (iSCs), in particular induced pluripotent stem (iPS) cells, derived from an mammal which suffers from at least one adverse effect caused by a medical treatment of a disease in said mammal, wherein said mammal has a genetic defect or genetic predisposition that is linked with said adverse effect against said medical treatment, comprising reprogramming of somatic cells obtained from said mammal with at least one suitable reprogramming factor. The present invention further relates to iSC-cell lines, preferably iPS-cell lines and their uses in screening methods in the context of searching for treatments lacking or having reduced side-effects.

In accordance with EC regulations, new and even already existing substances that are used pharmaceutically or industrially must be tested for toxicity. Currently, mainly animal testing is used since no acceptable in vitro testing protocols are available. In addition, also the development of substances as pharmaceuticals includes toxicity tests.

The current process of the development of medicaments is very inefficient and the chances for success are low: more than 90 % of the drugs as tested in the clinical phases finally can not be used. The main reasons for this high failure rate are found in an insufficient bioavailability and toxicity of the drug candidates (ADME/Tox Technologies, 2008).

Thus, current methods for developing drugs do not take into account these criteria at all or only insufficiently. If considering further that in the preclinical phase a large number of animal tests are required, the low efficiency presents a major problem not only in view of commercial but also in view of ethical aspects.

One limitation of the currently existing animal tests and *in vitro* test systems is that individual-specific differences, such as, for example, genetic variants that influence a different metabolization or compatibility of the substances are not taken into account. Problems that result from this become evident only during the clinical testing of phases II or III. For cell based test methods furthermore the problem exists that the cells that are employed are often not available in sufficient amounts and/or have a limited tissue specify.

The liver is a decisive organ for a multitude of metabolic processes and disease symptoms. Thus, a tissue specific human liver test system is of immense interest for different disciplines, such as, for example, toxicology, regenerative medicine, biology of infections, and physiology.

Test systems that mimic tissue specific properties of the liver are also of high commercial interest. They are desperately sought for in both the pharmaceutical drug development in the area of ADME/Tox as well as for substance tests for a risk classification of chemical substances. In 2007, the size of the world wide market for *in vitro* ADME/Tox was about 410 Mio. $.

Approaches to replace part of the animal tests with ethically indisputable, faster and cheaper cell-based in vitro tests, in case of hepatocytes, have failed, since primary hepatocytes (in particular human hepatocytes) are not available or are not available in the required amounts. In addition, hepatocytes in culture de-differentiate within a few days and thus do no longer exhibit important liver-specific properties. Primary animal hepatocytes do not reflect the specific human situation and, similar to hepatocyte-derived cell lines, do not represent an alternative.

EP 08 012 010.8 describes a simple technology for a controlled expansion of cells.

The following applications describe the in vitro differentiation of pluripotent stem cells into differentiated cells, based on embryonic stem cells. In WO 01/81549, human embryonic stem cells are allowed to form embryoid bodies, and then are combined with the differentiation agent n-butyrate, optionally supplemented with maturation factors. In another example, n-butyrate is added to human embryonic stem cells in feeder-free culture. A uniform cell population is obtained, which is predominated by cells with morphological features of hepatocytes, expressing surface markers characteristic of hepatocytes, and having enzymatic and biosynthetic activity important for liver function.

WO 2007/110966 describes a method for the preparation of hepatocytes using ES cells. Disclosed is a promoter for the differentiation of an ES cell, preferably a promoter for the differentiation of an ES cell into a hepatocyte, containing uPA or a prophylactic agent for teratoma. Also disclosed is a method for promoting the differentiation of an ES cell, preferably the dif ferentiation of an ES cell into a hepatocyte, comprising the step of contacting uPA with the ES cell, or a method for preparing a hepatocyte comprising the step of contacting uPA with an ES cell to cause the differentiation of the ES cell into a hepatocyte.

WO 2007/002568 describes reporter hepatocytes and other cells for drug screening and toxicity testing as a system for rapid determination of pharmacologic effects on target tissue types in cell populations cultured in vitro. The cells contain a promoter-reporter construct that reflects a toxicological or metabolic change caused by the agent being screened. The promoter is taken from a gene known to be up- or down-regulated according to the metabolic state of the cell, and linked to a reporter gene that provides an external signal for monitoring promoter activity. The promoter-reporter cells may be produced by placing these genetic alterations into a line of human embryonic stem cells, bulking up the cells to any extent desired, and then differentiating the cells into the desired tissue type.

WO 2007/143117 describes the differentiation of primate pluripotent cells into hepatocyte-lineage cells. In certain embodiments, the methods utilize sequential culturing of the primate pluripotent stem cells in certain growth factors to produce hepatocyte-lineage cells. In certain embodiments, the population of cells produced by the methods is further enriched for hepatocyte-lineage cells.

Finally, WO 2007/069666 describes a method for producing induced pluripotent stem cells (iPS), which comprises a step of contacting a nuclear reprogramming factor with the somatic cell. Said factor can be selected from a gene product of an Oct family gene, a Klf family gene, and a Myc family gene, and optionally a further a gene product of a Sox family gene, such as Sox2. Furthermore, a cytokine such as bFGF and/or SCF can be added. As additional factors, the TERT gene, SV40 Large T antigen, HPV16 E6, HPV16 E7, Bmil, Fbx15, Nanog, ERas, ECAT15-2, Tcl1, catenin, ECAT1, Esg1, Dnmt3L, EAT8, Gdf3, Sox15, ECAT15-1, Fthl17, Sall4, Rex1, UTF1, Stella, Stat3, and Grb2 are described.

Despite the above progress, an unsolved problem is that fact that in individuals, due to the patient specific genetic background (polymorphisms), drugs and medical treatments can be differently metabolized, and thus have different toxicities and adverse side-effects and/or are less effective. Test methods that take into account these complexities are currently not available, and these problems become apparent only in late clinical phases.

It is therefore an object of the present invention, to provide methods and products in order to help to address the above problems associated with adverse side-effects in the context of medical treatment of a disease or condition or the prevention thereof in a patient or patient group with a specific genetic background. Other objects and advantages of the present invention will become apparent upon reading the following detailed description thereof.

In a first particularly preferred aspect of the present invention, the object of the invention is solved by an *in vitro* method for producing induced stem cells (iSCs) derived from a mammal which suffers from at least one adverse effect caused by a medical treatment of a disease or medical condition or the prevention thereof in said mammal, wherein said mammal has a genetic defect or genetic predisposition that is linked with said adverse effect against said medical treatment of a disease or medical condition or the prevention thereof, comprising reprogramming of somatic cells obtained from said mammal with at least one suitable reprogramming factor, with the provisio that totipotent human stem cells are excluded. Preferred is a method according to the above, wherein said iSC is an induced pluripotent stem (iPS) cell.

In a general scheme, induced pluripotent stem (iPS) cells are generated by (1) isolating and culturing donor cells. (2) Transfecting stem cell-associated genes into the cells by viral vectors, and identifying cells expressing the exogenous genes. (3) Harvesting and culturing the cells according to ES cell culture, using mitotically inactivated feeder cells. (4) A small subset of the transfected cells will become iPS cells and generate ES-like colonies.

A particularly preferred further aspect of the present invention relates to a method that combines the steps of generating iPS, the differentiation thereof, and a conditional expansion of the cells as described herein. Through selectively combining these steps, for the first time the problems of the methods of the state of the art are elegantly overcome, in particular the production and expansion of homogeneous test cells with a specific genetic background in sufficient amounts.

In a second particularly preferred aspect of the present invention, the object of the invention is solved by providing an iSC-cell line, preferably an iPS-cell line that is produced based on a method according to the present invention.

There have been recent attempts to generate specifically differentiated cell types from embryonic stem cells. Due to the development of the iPS technology, it is now possible to generate stem cells from patients through de-differentiation, without the ethical problems involved in other stem cell based approaches, and to use said cells for the development of cell culture based and other assay systems as described herein. The further development of in vitro differentiation protocols provides a broad variety of different new cell types and assay systems derived therefrom.

Nevertheless, the approaches are yet limited by the very proliferative activity of the differentiated cells. The object of the present invention is based on the problem that individual specific reactions to medications or other treatments can not be addressed using current in vitro assay methods, and are not sufficiently covered by respective animal models. According to the invention the singular, or in preferred embodiments combined, use of patient-group specific iPSs together with specific differentiation protocols using the initiation of the proliferation allows for the production and expansion of homogeneous test cells with a specific genetic background.

The present invention thus addresses the problem of the individual specificity, in that iPS are produced from individuals that show genetically related side-effects in view of the acceptance of a medical treatment of a disease or condition. For the purposes of the present invention, it is generally irrelevant, whether the defects are characterized in detail or not. Cell banks can be produced from the iPSs of the genetically diverse donors that allow for the production of test cells through the in vitro differentiation. Hereby, differentiation protocols of different kinds can be used in order to obtain test cells showing the suitable differentiation. The expansion of these cells is achieved through the induction of a controlled proliferation.

Clarke and Haselden (in: JN.Clarke CJ, Haselden JN. Metabolic profiling as a tool for understanding mechanisms of toxicity. Toxicol Pathol. 2008;36(1):140-7) describe the concept of metabolic profiling (metabolomics/metabonomics) as the measurement in biological systems of the complement of low-molecular-weight metabolites and their intermediates that reflects the dynamic response to genetic modification and physiological, pathophysiological, and/or developmental stimuli. As an example, the measurement and interpretation of the endogenous metabolite profile from a biological sample (typically urine, serum, or biological tissue extract) is described as providing many opportunities to investigate the changes induced by external stimuli (e.g., drug treatment) or enhance the knowledge of inherent biological variation within subpopulations. The article explains the basic principles of metabolic profiling and how the tools (nuclear magnetic resonance [NMR], liquid chromatography-mass spectrometry [LC-MS]) can be applied in toxicology and pathology. Metabolic profiling can complement conventional methodologies and other "omics" technologies in investigating preclinical drug development issues. A key message is that metabolic profiling offers huge potential to highlight biomarkers and mechanisms in support of toxicology and pathology investigations in preclinical drug development.

Another preferred aspect of the present invention then relates to the *in vitro* method for producing induced stem cells (iSCs) derived from an mammal which suffers from at least one adverse effect caused by a medical treatment of a disease or medical condition or the prevention thereof in said mammal, wherein said mammal is selected from mice, rats, monkeys, pigs, dogs, cats, cows, sheep, goats, horses, guinea pigs, and, preferably, humans.

Another preferred aspect of the present invention then relates to the method according to the present invention as above, wherein said adverse effect against said medical treatment of a disease or medical condition or the prevention thereof is selected from hepatotoxicity, transplant rejection, allergic reactions, weakness of the immune system, cancer, pain, fever, rash, sleep-wake-pattern, seizures, vertigo, nausea, delayed wound healing, blood clotting disorders, hyperactivity, and lack of bioavailability. Other adverse effects can occur as a collateral or side effect of many interventions, but they are particularly important in pharmacology, due to its wider, and sometimes uncontrollable, use by way of self-medication. Thus, responsible drug use becomes an important issue here. Adverse effects, like therapeutic effects of drugs, are a function of dosage or drug levels at the target organs, so they may be avoided or decreased by means of careful and precise pharmacokinetics, the change of drug levels in the organism in function of time after administration. Furthermore, most drugs have a large list of non-severe or mild adverse effects which do not rule out the interruption of usage. These ef fects have widely variable incidence, according to individual sensitivity. They comprise nausea, dizziness, diarrhea, malaise, vomit, headache, dermatitis, dry mouth, etc. In the context of the present invention, said adverse effect against said medical treatment of a disease or medical condition or the prevention thereof is based on or linked with a genetic defect or genetic predisposition of the mammal that is treated.

As preferred examples of the method according to the present invention, said genetic defect or genetic predisposition that is linked with said adverse effect is induced by external stimuli, such as, for example, said medical treatment of a disease or medical condition (such as treatment with a mutagenic substance), or is present as inherent biological variation within a subpopulation of said mammal, such as a mutation, deletion, duplication, and/or SNP-related variation, in particular the polymorphisms CyP2C9; SNPs in N-acetyl transferases, such as Nat2; SNPs in thiopurine-S-methyl transferase (TPMT), microsomal triglyceride transfer protein (MTTP)-I128T polymorphism, SNPs rs3772622, rs3772633, rs2276736, rs3772630, rs3772627, and SNP rs3792323.

In general, all suitable somatic cells can be used for the methods of the present invention. In principle, cells are used that are capable of reprogramming using the methods as described herein. Another preferred aspect of the present invention then relates to the method according to the present invention as above, wherein said somatic cells obtained from said mammal are selected from somatic fibroblasts, endothelial cells, and hematopoietic precursor cells.

Yet another preferred aspect of the present invention then relates to the method according to the present invention as above, wherein said reprogramming comprises contacting a nuclear reprogramming factor with said somatic cell, wherein said factor is preferably selected from a selected from a protein, a gene expressing said protein, a regulatory RNA, and a chemical substance, and more preferably selected selected from at least one gene product selected from an Oct family gene, a Klf family gene, and a gene product of a Sox family gene, preferably Oct4, Sox2, and Klf4, and, optionally, a Myc family gene, and preferably c-Myc.

The generation of iPS cells is crucial on the genes used for the induction. Oct-3/4 and certain members of the Sox gene family (Sox1, Sox2, Sox3, and Sox15) have been identified as crucial transcriptional regulators involved in the induction process whose absence makes induction impossible. Additional genes, however, including certain members of the Klf family (Klfl, Klf2, Klf4, and Klf5), the Myc family (C-myc, L-myc, and N-myc), Nanog, and LIN28, have been identified to increase the induction efficiency.

Oct-3/4 (Pou5f1): Oct-3/4 is one of the family of octamer ("Oct") transcription factors, and plays a crucial role in maintaining pluripotency. The absence of Oct-3/4 in Oct-3/4⁺ cells, such as blastomeres and embryonic stem cells, leads to spontaneous trophoblast differentiation, and presence of Oct-3/4 thus gives rise to the pluripotency and differentiation potential of embryonic stem cells.

The Sox family of genes is associated with maintaining pluripotency similar to Oct-3/4, although it is associated with multipotent and unipotent stem cells in contrast with Oct-3/4, which is exclusively expressed in pluripotent stem cells. While Sox2 was the initial gene used for induction by Yamanaka et al., Jaenisch et al., and Thompson et al., other genes in the Sox family have been found to work as well in the induction process. Sox1 yields iPS cells with a similar efficiency as Sox2, and genes Sox3, Sox15, and Sox18 also generate iPS cells, although with decreased efficiency.

Klf4 of the Klf family of genes was identified as a factor for the generation of mouse iPS cells and was demonstrated as a factor for generation of human iPS cells. There are reports, nevertheless, that Klf4 was unnecessary for generation of human iPS cells and in fact failed to generate human iPS cells. Klf2 and Klf4 were found to be factors capable of generating iPS cells, and related genes Klfl and Klf5 did as well, although with reduced efficiency.

The Myc family of genes are proto-oncogenes implicated in cancer. It was demonstrated that c-myc is a factor implicated in the generation of mouse iPS cells and Yamanaka et al. demonstrated it was a factor implicated in the generation of human iPS cells. Thomson et al., Yamanaka et al., and unpublished work by Johns Hopkins University reported that c-myc was unnecessary for generation of human iPS cells. Usage of the "myc" family of genes in induction of iPS cells is troubling for the eventuality of iPS cells as clinical therapies, as 25% of mice transplanted with c-myc-induced iPS cells developed lethal teratomas. N-myc and L-myc have been identified to induce instead of c-myc with similar efficiency.

In another preferred aspect, the method according to the present invention can be performed through a reprogramming that further comprises the addition, and preferably expression, of at least one additional factor, such as a protein, a gene expressing said protein, the variation of culture conditions, and/or a chemical substance, and preferably Nanog. Using these additional factors, mainly the efficiency of the processes can be improved. For example, in embryonic stem cells, Nanog, along with Oct-3/4 and Sox2, is necessary in promoting pluripotency. Nevertheless, it is possible to generate iPS cells with Nanog as one of the factors. As another example, LIN28 is an mRNA binding protein expressed in embryonic stem cells and embryonic carcinoma cells associated with differentiation and proliferation. Thomson et al. demonstrated it is a factor in iPS generation, although it is unnecessary.

Preferred is a method according to the present invention, wherein said nuclear reprogramming factor is recombinantly introduced into said somatic cell through a genetic expression construct, such as a retroviral expression construct, preferably an adenoviral construct, wherein said genetic expression can be preferably induced by adding an inducing factor, such as doxycycline. Other inducing factors can also be used, such as tetracycline or neomycin. It is further preferred that the expression is strictly inducing factor dependent, and can thus be switched on or off depending from the presence of absence of the factor.

Preferred is a method according to the present invention, further comprising the step of selecting and/or expanding the iSCs expressing said nuclear reprogramming factors, and preferably generate ES-like colonies. This is preferably based on the expansion vectors as described herein. The cells as infected are cultivated and expanded in the presence of, for example, the inductor doxycycline (Dox). The expanded cultures were characterized regarding the regulation of the expansion genes, their proliferation in the presence and absence of Dox and the expression of, for example, relevant liver markers, such as, for example, c-met, myc, hTert, and β-catenin. Furthermore, binary vectors for a liver specific activation of ubiquitary expansion cassettes are constructed. For this, the trans-activator is activated in a liver specific manner through defined promoters, and a tTA dependent activation of the expansion genes is achieved. Vectors are obtained that allow for a co-transduction of the binary expression cassette.

Further preferred is a method according to the present invention, further comprising the step of identifying said iSCs, and preferably said iPSs, through analytical methods such as teratome formation experiments, qRT-PCR for markers of pluripotency, and/or chimerism analyses following blastocyst-injection. 14 to 18 days past retroviral transduction, suitable ES-like colonies were subcloned according to morphologic criteria, and cultivated as iPS-lines.

Then, further preferred is a method according to the present invention, further comprising the step of a reversion of the expression of the factors as expressed, preferably through conditional silencing of the expression, excision of said factors, for example through a cre/lox system, and/or genetic silencing of said factors. It is further preferred that the expression is strictly inducing factor dependent (as described above), and can thus be switched on or off depending from the presence of absence of the factor.

Even further preferred is a method according to the present invention, further comprising the step of differentiating said iSCs, and preferably said iPSs, into cells of a desired phenotype, preferably neuronal cells, cardiomyocytes, hepatocytes, and bone marrow cells, comprising, for example, the use of instructive cytokines, such as, for example, activin A, BMP4, bFGF, VEGF, Wnt, and/or Notch. iPSCs are capable of differentiation in a fashion similar to ESCs into fully differentiated tissues, as shown by the following examples.

Still even further preferred is a method according to the present invention, wherein said cells are (and/or have been) transduced with a selection construct, harboring a phenotype-specific, preferably a neuronal cell-, cardiomyocyte-, hepatocyte-, or bone marrow cell-specific promoter controlling marker proteins, such as GFP, GFP-variants or antibiotic-resistance genes, such as neomycin and/or puromycin.

Neural Differentiation: iPSCs were differentiated into neurons, expressing βIII-tubulin, tyrosine hydroxylase, AADC, DAT, ChAT, LMX1B, and MAP2. The presence of catecholamine-associated enzymes may indicate that iPSCs, like hESCs, may be differentiable into dopaminergic neurons. Stem cell-associated genes were downregulated after differentiation. Cardiac Differentiation: iPSCs were differentiated into cardiomyocytes that spontaneously began beating. Cardiomyocytes expressed TnTc, MEF2C, MYL2A, MYHCβ, and NKX2.5. Stem cell-associated genes were downregulated after differentiation.
Teratoma Formation: iPSCs injected into immunodeficient mice spontaneously formed teratomas after nine weeks. Teratomas are tumors of multiple lineages containing tissue derived from the three germ layers endoderm, mesoderm and ectoderm; this is unlike other tumors, which typically are of only one cell type. Teratoma formation is a landmark test for pluripotency.
Embryoid Body: hESCs in culture spontaneously form ball-like embryo-like structures termed "embryoid bodies", which consist of a core of mitotically active and differentiating hESCs and a periphery of fully differentiated cells from all three germ layers. iPSCs also form embryoid bodies and have peripheral differentiated cells.
Blastocyst Injection: ESCs naturally reside within the inner cell mass (embryoblast) of blastocysts, and in the embryoblast, differentiate into the embryo while the blastocyst's shell (trophoblast) differentiates into extraembryonic tissues. The hollow trophoblast is unable to form a living embryo, and thus it is necessary for the embryonic stem cells within the embryoblast to differentiate and form the embryo. iPSCs were injected by micropipette into a trophoblast, and the blastocyst was transferred to recipient females. Chimeric living mouse pups were created: mice with iPSC derivatives incorporated all across their bodies with 10%-90% chimerism.

Reporter constructs were stably introduced into the murine iPS-lines, and from these cells, iPS-derived liver-precursor cells were generated as an example. One of the proteins that is highly expressed in the adult liver is albumin. Therefore, reporter constructs under the transcriptional albumin-promoter control are suitable for a selection of hepatic cells. A lentiviral reporter construct was constructed that expressed EGFP under the control of the albumin promoter (Alb-EGFP). A similar construct with a neomycin-resistance cassette (Alb-Neo), and a construct with both markers connected through an *internal ribosomal entry site* (IRES) (Alb-EGFP-IRES-Neo) were produced.

For the identification of early liver precursors an additional marker was established. Alphafetoprotein (AFP) is also a highly expressed protein in early hepatic cells. Since this marker is also expressed in the extra-embryonic endoderm, another marker was used for isolating early hepatic precursor cells. Thus, a Sox17-promoter controlled construct with the red fluorescent protein (Sox17-RFP) was generated in order to be able to perform double selections. Sox17 (SRY-box containing gene 17) is an essential transcription factor for the formation of the definitive endoderm, and is absent in derivatives of the primitive endoderm.

Further preferred is a method according to the present invention, wherein said method further comprises subjecting said cells of a desired phenotype to one or more factors or conditions controlling the proliferation of said cells, such as a) defined culture conditions, b) cytokines, such as bFGF, c) expression of recombinant expression of genes such as c-myc, and/or SCF, TERT, SV40 Large T antigen, HPV16 E6, HPV16 E7, Bmil, Fbx15, ERas, ECAT15-2, Tcl1, catenin, ECAT1, Esg1, Dnmt3L, EAT8, Gdf3, Sox15, ECAT15-1, Fthl17, Sal14, Rex1, UTF1, Stella, Stat3, and Grb2, d) expression of regulatory RNAs, and/or e) application of proliferation modifying/controlling chemical substances.

According to the present invention, the factor(s) is (are) suitably introduced into said somatic cell. Preferred is a recombinant introduction into said somatic cell through a genetic expression construct, such as through a retroviral expression construct, preferably an adenoviral construct, wherein said genetic expression can be preferably induced by adding an inducing factor, such as doxycycline and/or excised, such as through a cre/lox system.

Yet another preferred aspect of the present invention then relates to an (isolated) iSC-cell line, preferably an iPS-cell line, produced according to a method according to the present invention as described herein. These cell lines form the material for a variety of uses, such as diagnostic or screening tools, as will be explained further. Yet another preferred aspect of the present invention then relates to a (isolated) transgenic mammalian cell line, produced according to a method according to the present invention as described herein.

Yet another preferred aspect of the present invention then relates to a transgenic non-human mammal, comprising a cell line according to the present invention. Again, this mammal can be used for a variety of uses, such as diagnostic or screening assays.

In another preferred aspect of the present invention, the cell-lines or non-human mammals as produced can be used to screen for factors (such as solvents, small molecule drugs, peptides, oligonucleotides) that affect the characteristics of such cells and their progeny. Certain methods of screening are known in the art and are discussed, e.g., in In vitro Methods in Pharmaceutical Research, Academic Press, 1997; and in U.S. Patent 5,030,015.

In another preferred aspect thereof, the present invention relates to an *in vitro* or *in vivo* method for diagnosing (or screening) for at least one adverse effect caused by a medical treatment of a disease or medical condition or the prevention thereof in a mammal, comprising the steps of: a) providing an iSC-cell line, preferably an iPS-cell line according to the present invention, or a transgenic mammalian cell line according to the present invention, or a transgenic non-human mammal according to the present invention, b) contacting said cell line or mammal with a candidate compound, either alone or in combination with at least one additional compound, c) determining if the cells or the mammal undergo any change in morphology, marker expression, or functional activity being attributable to said compound, compared to a cell line derived from a mammal lacking said genetic defect or genetic predisposition that is linked with said adverse effect against said medical treatment of a disease or medical condition or the prevention thereof, and d) concluding on said at least one adverse effect caused by said medical treatment of a disease or medical condition or the prevention thereof in said mammal.

In this assay, the cells of the present invention are diagnosed with pharmaceutical compounds for additional pharmacological side-effects on, for example, a hepatocyte-lineage cells. In preferred embodiments, pharmaceutical compounds are screened using, for example, hepatocyte cells to determine whether compounds designed to have effects elsewhere have unintended side effects on hepatocyte-lineage cells.

In another preferred aspect thereof, the present invention then relates to an *in vitro* or *in vivo* method for screening for a compound for a medical treatment of a disease or medical condition or the prevention thereof in a mammal lacking or having reduced side-effects, comprising the method for diagnosing for at least one adverse effect caused by a medical treatment of a disease or medical condition or the prevention thereof in a mammal as above, wherein no change in morphology, marker expression, or functional activity being attributable to said compound is indicative for a treatment lacking or having reduced side-effects. In this embodiment, pharmaceutical compounds are screened using, for example, hepatocyte cells or mammals inn order to determine whether compounds designed to have effects elsewhere have unintended/unwanted side effects on hepatocyte-lineage cells.

In yet another preferred aspect thereof, the present invention then relates to an *in vitro* or *in vivo* method for screening for a compound reverting or reducing at least one adverse effect caused by a medical treatment of a disease or medical condition or the prevention thereof in a mammal, comprising the method for diagnosing for at least one adverse effect caused by a medical treatment of a disease or medical condition or the prevention thereof in a mammal as above in the presence of at least one additional compound in step b), wherein no change in morphology, marker expression, or functional activity being attributable to said additional compound in step b) is indicative for a compound reverting or reducing at least one adverse effect caused by said medical treatment of a disease or medical condition or the prevention thereof.

In certain embodiments, assessment of the activity of candidate pharmaceutical compounds involves combining the cells or the mammal with a candidate compound, either alone or in combination with other compounds. An investigator may then determine whether the cells have undergone any change in morphology, marker expression, or functional activity that may be attributable to the compound. Untreated cells or mammals may be used as controls.

Cytotoxicity can be determined, in various embodiments, by the effect on cell viability, survival, morphology, and/or the expression of certain markers and receptors. In addition, effects of a drug on chromosomal DNA can be determined, e.g., by measuring DNA synthesis or repair. 3H-thymidine or BrdU incorporation, especially at unscheduled times in the cell cycle, or above the level required for cell replication, may be consistent with a drug effect. Unwanted drug effects can also include unusual rates of sister chromatid exchange, which may be determined, e.g., by metaphase spread. See, e.g., A. Vickers, pp 375-410 in In vitro Methods in Pharmaceutical Research, Academic Press, 1997.

Drug effects on cell function can be assessed, e.g., using standard assays to observe phenotype and/or activity of cells. Such activities include, but are not limited to, marker expression, receptor binding, and enzymatic activity. Certain activities that can be assayed in order to evaluate, for example, hepatotoxicity include, but are not limited to, synthesis and/or secretion of albumin, cholesterol, and/or lipoproteins; transport of conjugated bile acids and/or bilirubin; ureagenesis; cytochrome p450 levels and/or activities; glutathione levels; release of alpha-glutathione s-transferase; ATP, ADP, and/or AMP metabolism; intracellular K+ and/or Ca2+ concentrations; release of nuclear matrix proteins and/or oligonucleosomes; and induction of apoptosis (indicated by, e.g., cell rounding, condensation of chromatin, and/or nuclear fragmentation). Where an effect is observed, the concentration of the compound can be titrated to determine the median effective dose (ED50).

In another aspect of the present invention, the compound lacking or having reduced side-effects and/or said compound reverting or reducing at least one adverse effect caused by said medical treatment of a disease or medical condition or the prevention thereof can be formulated into a pharmaceutical composition in a method for producing a pharmaceutical composition, comprising a method for screening as above, and formulating said compound lacking or having reduced side-effects and/or said compound reverting or reducing at least one adverse effect caused by said medical treatment of a disease or medical condition or the prevention thereof together with a pharmaceutically acceptable carrier, excipient, and/or stabilizer.

Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

In yet another preferred aspect thereof, the present invention then relates to a method of treatment of a disease, comprising providing to a patient in need thereof a pharmaceutical composition produced according to the invention as above. In the context of the present invention, said treatment of a disease can be selected from a method wherein an adverse effect against said medical treatment of a disease or medical condition or the prevention thereof is selected from hepatotoxicity, neurotoxicity, transplant rejection, allergic reactions, weakness of the immune system, cancer, pain, fever, rash, sleep-wake-pattern, seizures, vertigo, nausea, delayed wound healing, blood clotting disorders, hyperactivity, and lack of bioavailability. That is, a disease is treated that involves an adverse effect against said medical treatment of a disease or medical condition or the prevention thereof. Here, for example, the compound as administered counteracts against the adverse effect, and thus preferably improves the compliance of said treatment, and/or allows for higher doses to be employed of an otherwise not acceptable medically active agent. Further in the context of the present invention, said treatment of a disease can be selected from a method wherein the actual disease to be treated is selected from liver diseases, proliferative disorders, such as cancer, inflammatory diseases, graft-versus-host reactions, viral or bacterial infections, trauma, depression, diabetes, obesity, allergic reactions, and stroke.

In yet another preferred aspect thereof, the present invention then relates to the use of a pharmaceutical composition produced according to the present invention for the treatment of an adverse effect against a medical treatment of a disease or medical condition or the prevention thereof, wherein said adverse effect is preferably selected from hepatotoxicity, neurotoxicity, transplant rejection, allergic reactions, weakness of the immune system, cancer, pain, fever, rash, sleep-wake-pattern, seizures, vertigo, nausea, delayed wound healing, blood clotting disorders, hyperactivity, and lack of bioavailability. Here, just like in the above methods of treatment, the compound as administered counteracts against the adverse effect, and thus preferably improves the compliance of said treatment, and/or allows for higher doses to be employed of an otherwise not acceptable medically active agent. Another preferred aspect of the present invention then relates to the use of a pharmaceutical composition produced according to the present invention for the treatment of a disease selected from liver diseases, proliferative disorders, such as cancer, inflammatory diseases, graft-versus-host reactions, viral or bacterial infections, trauma, depression, diabetes, obesity, allergic reactions, and stroke, wherein said treatment lacks or has reduced side-effects.

In certain preferred embodiments, the, for example, hepatocyte cells provided herein can be used to enhance tissue maintenance or repair of liver tissue for any perceived need, including, but not limited to, liver tissue having an inborn error in metabolic function leading to side effects, liver tissue damaged by the side effect of a treatment of a disease or condition, liver tissue damaged by the effect of a toxin, and/or liver tissue damaged by trauma.

The present invention is based on a new strategy for generating cellular assay systems. Hereby, in the preferred embodiment, hepatic cells are generated from pluripotent stem cells as unlimited source of cells. The differentiation protocols as established that are based on induced pluripotent stem cells allow for the generation of multipotent liver precursor cells (PCs). Nevertheless, in these protocols the yield during the differentiation is low, and the differentiated precursor cells show only a low potential for proliferation. Therefore, the present invention combines the differentiation technology with a conditional expansion technology which was developed in the context of other cellular types.

The principle of the approach makes it possible, to produce human cell lines with (preferably defined) metabolic defects (e.g. based on polymorphisms) and thus to open up a new dimension in the testing of substances which is not available yet, neither using animal testing nor in first clinical tests. These cell lines also serve as a basis for assay and screening methods in order to evaluate and/or limit side-effects of chemical substances used in medicaments and therapy. In addition to measuring the hepatotoxicity, a particular focus is put on the metabolic properties and characteristics of the cells.

Currently, toxicological studies must largely be performed in animals. The reason for this is that liver cells already loose their functional properties after a few days in culture. Thus, the immortal hepatic cell lines as currently available do no longer exhibit the relevant liver specific markers and properties. There are first reports that three-dimensional culture conditions maintain some liver specific properties over several weeks. Nevertheless, a substantial expansion of the cells does not take place.

Hepatic cell lines were either isolated from tumor tissue or generated through the introduction of constitutively expressed immortalizing genes. Nevertheless, it was found that these cell lines (as well as cell lines of other differentiation stages) do not or only in a very low fraction reflect the properties of the original tissue. This is presumably due to the permanent expression of the tumor genes. Thus, they can not be used in many cases, and in particular for the testing of a metabolization of drugs and their toxicities.

In summary, in the present invention in one preferred embodiment thereof liver cells are generated as a surrogate for in vivo toxicity tests in the context of the initial screening for new drug substances for a complete replacement of animal testing. The few substances that do not show a significant in vitro toxicity, would then be tested in a much smaller scale in vivo, which leads to a reduction of the animal experiments as still deemed necessary. In particular, the overall burden of the remaining test animals is markedly reduced, since the particularly toxic substances are already ruled out in the initial screening, and are therefore never used in vivo.

In addition to the characterization of pluripotent stem cells in particular the hepatic differentiation potential of pluripotent stem cells could be analyzed. Currently, some *in vitro* differentiation protocols are published that can be used in order to generate hepatic precursor cells from undifferentiated pluripotent stem cells (Sharma AD, Cantz T, Vogel A, Schambach A, Hairdass D, Iken M, Bleidißel M, Manns MP, Schöler HR, Ott M. Murine Embryonic Stem Cell-Derived Hepatic Progenitor Cells Engraft in Recipient Livers with Limited Capacity of Liver Tissue Formation. Cell Transplantation, 2008; 17:313-323). These differentiation protocols can also be used with murine pluripotent stem cells that are obtained by means of parthenogenesis (Cantz, T., Han, D. W., Sebastiano, V., Bleidissel, M., and Schöler, H. Reprogramming potential of murine parthenogentic embryonic stem cells. Regenerative Medicine, 2007; 2(6) Suppl: S4) and with pluripotent cells that are reprogrammed by means of cell fusion (Cantz T, Bleidiße1 M, Stehling M, Schöler HR. In vitro differentiation of reprogrammed murine somatic cells into hepatic precursor cells. Biol Chem, 2008; 389:889-896). For the latter, similarly to normal ESC-derived hepatocyte-cells, a successful engraftment in a mouse model metabolic of liver diseases could be shown (Cantz T, Manns MP, Ott M. Stem Cells in Liver Regeneration and Therapy. (Review) Cell & Tissue Research, 2007; 331(1): 271-282).

More importantly, using the new technology for the generation of induced pluripotent stem cells from fibroblasts and other somatic cells for the first time disease-specific pluripotent stem cells can be used in order to examine the pathophysiological relevant phenotype of these diseases, for which no suitable cell culture model is available. In the context of the present invention, iPS-cells of a murine tyrosinemia-model (FAH^{-/-}-mice) and of a M. Wilson model (toxic milk mice) were generated, and their metabolic phenotypes can now be examined *in vitro.*

The inventors also recently established a conditional method for immortalization that is based on the expression of strictly doxycycline (dox)-dependent immortalizing genes through vector that encode for auto-regulated positive feedback modules (May T, Müller PP, Weich H, Froese N, Deutsch U, Wirth D, Hauser H (2005): Establishment of murine cell lines by constitutive and conditional immortalization. J. Biotech. 120 (1), 99-110. May, T. Hauser, H., Wirth, D., Müller, P.P. (2005). Interference of temperature and action of SV40 large T antigen in conditional immortalization. Biol. Biochem. Res. Comm. 327, 734-741, May T, Hauser H. and Wirth D. (2007) In vitro expansion of tissue cells by conditional proliferation. Methods in Molecular Biology, 140:1-15.: 1-15). Immortalized cells grow strictly Dox-dependent and can be used for the expansion of primary cells. In contrast to unregulated systems (constitutive expression of the immortalizing genes), the expression of the immortalizing genes can be reverted. Using RNA-profiling studies it could be shown that that the dox-induced changes can be completely reversed (May, T., Hauser, H., Wirth, D. (2004): Transcriptional control of SV40 T-antigen expression allows a complete reversion of immortalization. Nucleic Acids Res., 32, 5529-5538).

It could be furthermore shown that the auto-regulated modules lead to a strictly binary expression and at the same time can be efficiently transduced in a single step using lentiviral gene transfer (May, T., L. Eccleston, S. Herrmann, H. Hauser, J.Goncalves, and D.Wirth. 2008. Bimodal and hysteretic expression in mammalian cells from a synthetic gene circuit. PLoS.ONE. 3: e2372). Respective vectors were successfully used for a transduction und controlled expression of different immortalizing genes. This allowed for the immortalization of cells of different species and differentiation statuses: In addition to fibroblasts, also endothelial cells from human and mouse were immortalized (May, T., Hauser, H., Wirth, D. (2004): Transcriptional control of SV40 T-antigen expression allows a complete reversion of immortalization. Nucleic Acids Res., 32, 5529-5538).

It was shown in the context of the present invention, that even hematopoietic precursor cells can be immortalized using these vectors, while maintaining the stem cell markers.

In contrast to the use of broad and unspecific immortalizing genes the present approach is preferably based on the use of differentiation specific endogenous expansion genes. This reduces the probability of irreversible damages in the cells.

The present invention established methods for generating iPSs, in particular hepatic precursor cells (PCs). Furthermore, specific hepatic precursor cell lines are established. These cell lines have differentiation properties that are very close to freshly isolated primary hepatic cells. These cell lines are of particularly interest in view of several aspects.
1) Scientifically: A scientific system is provided that due to its high similarity to the *in vivo* situation for the first time offers the possibility to examine a series of questions with unlimited cell systems. In that, for example, liver cells are produced from individual persons and/or specific subpopulations using induced pluripotent stem cells (iPS), a pre-screening regarding the pharmaceutical acceptability in "normal" and individuals is given that have genetic polymorphisms in view of, for example, the liver metabolism.
2) Commercially: An *in vitro* test system is provided to the chemical and pharmaceutical industry that, compared with the animal models as currently used, is ethically less problematic, cheaper, and faster, and is more efficient in evaluating the pharmaceutical acceptability of the substances as tested.
3) Ethical aspects: Currently, the toxicity studies for the evaluation of pharmaceutical drug candidates make up for about 4% of all animal tests per year in Europe. This means that nearly half a million rats and mice are used for toxicity studies. Using the cell lines and the assays according to the present invention, this number will be reduced drastically.

The present invention will now be described in more detail in the following preferred examples, nevertheless, without being limited thereto. For the purposes of the present invention, all references as cited are incorporated in their entireties. In the Figures;

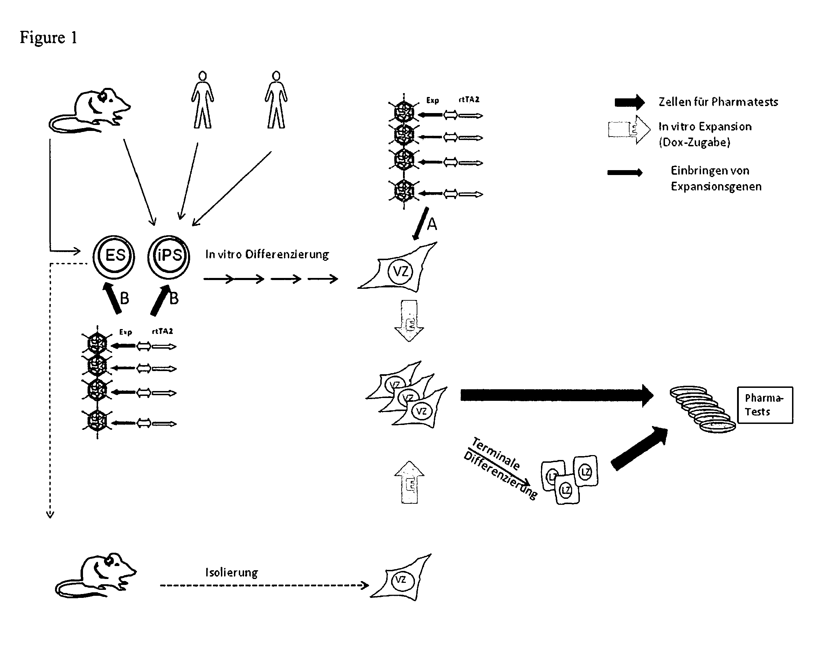

Figure 1 shows a schematic representation of the different steps of the production of cells, starting from embryonic stem cells (ES) or iPS. ES are prepared from mice or iPS (as used for the present invention) are prepared from mice or other mammals, such as humans, and rendered into, for example, hepatocyte precursor-cells (PCs) through in vitro differentiation. For the expansion of these cells, different cassettes are stably integrated (red arrow) into the ES/iPS and/or PCs using viral transfer, whereby two different targets are used (A, B). In response to external stimuli (doxycycline; E) the proliferation is stimulated through the activation of the gene cassettes. Thereby, an expansion of the cultures is achieved (only as long as the stimulator is present in the medium). The cells then can be used directly in *in vitro* tests, such as screening tests, or terminally differentiated and then used in said tests. An alternative (bottom) results from the production of transgenic mice from the ES cells with the expansion cassette. From this, again precursor-cells can be isolated and conditionally brought to proliferation and expansion through a stimulation of the gene cassettes. Furthermore the expanded PCs are terminally differentiated.

### Examples

The following examples are related to the preferred generation of liver cells in order to generate a test model for the testing of hepatotoxicity of medicaments for liver cells in particular genetic backgrounds. Nevertheless, as described above, the person of skill will be readily able to adjust the following examples to the generation of other cell types, such as bone marrow cells, neurons, myocytes and other desired types of cells.

### 1. Generation of liver precursor cells from iPS in mice

Several murine iPS lines have been established, based on a wild type background (C3H, C57BL/6, and Balb/c) or from mouse models with metabolic liver diseases. For this, retroviral constructs for the expression of Oct4, Sox2, Klf4, and, optionally, c-Myc were used.

14 to 18 days past retroviral transduction, suitable ES-like colonies were subcloned according to morphologic criteria, and cultivated as iPS-lines. For all established lines, the pluripotency is confirmed by means of teratoma formation experiments, by means of qRT-PCR for pluripotency-markers, and by means of chimerism-analyses following blastocyst-injection.

### 2. Introduction of selection-constructs and hepatic differentiation of the iPS-cells Reporter constructs were stably introduced into the murine iPS-lines, and from these cells, iPS-derived liver-precursor cells were generated as mentioned above.

One of the proteins that is highly expressed in the adult liver is albumin. Therefore, reporter constructs under the transcriptional albumin-promoter control are suitable for a selection of hepatic cells. A lentiviral reporter construct was constructed that expressed EGFP under the control of the albumin promoter (Alb-EGFP). A similar construct with a neomycin-resistance cassette (Alb-Neo), and a construct with both markers connected through an *internal ribosomal entry site* (IRES) (Alb-EGFP-IRES-Neo) were produced.

For the identification of early liver precursors an additional marker was established. Alphafetoprotein (AFP) is also a highly expressed protein in early hepatic cells. Since this marker is also expressed in the extra-embryonic endoderm, another marker was used for isolating early hepatic precursor cells. Thus, a Sox17-promoter controlled construct with the red fluorescent protein (Sox17-RFP) was generated in order to be able to perform double selections. Sox17 (SRY-box containing gene 17) is an essential transcription factor for the formation of the definitive endoderm, and is absent in derivatives of the primitive endoderm.

### 3. Generation of liver precursor cells from iPS in humans

In analogy to the above cells from mice, human iPS-derived liver cells were generated. For this, again the retroviral constructs for at least one of Oct4, Sox2, Klf4, and optionally c-Myc are used. Alternatively, other methods can be used, such as, for example, described in Woltjen et al. (Woltjen K, Michael IP, Mohseni P, Desai R, Mileikovsky M, Hämäläinen R, Cowling R, Wang W, Liu P, Gertsenstein M, Kaji K, Sung HK, Nagy A. piggyBac transposition reprograms fibroblasts to induced pluripotent stem cells. Nature. 2009 Mar 1, and Kaji K, Norrby K, Paca A, Mileikovsky M, Mohseni P, Woltjen K. Virus-free induction of pluripotency and subsequent excision of reprogramming factors. Nature. 2009 Mar 1.), and Kim et al. (exemplified with neural cells, Kim JB, Sebastiano V, Wu G, Araúzo-Bravo MJ, Sasse P, Gentile L, Ko K, Ruau D, Ehrich M, van den Boom D, Meyer J, Hübner K, Bernemann C, Ortmeier C, Zenke M, Fleischmann BK, Zaehres H, Schöler HR. Oct4-induced pluripotency in adult neural stem cells. Cell. 2009 Feb 6;136(3):411-9.), and Sridharan R, Tchieu J, Mason MJ, Yachechko R, Kuoy E, Horvath S, Zhou Q, Plath K. Role of the murine reprogramming factors in the induction of pluripotency. Cell. 2009 Jan 23;136(2):364-77. Furthermore, the technology will be transferred to human iPS from donors having relevant disease-related polymorphisms (e.g., and preferred, CyP2C9; N-actetyl transferases, such as, Nat2; thiopurine-S-methyl transferase (TPMT), microsomal triglyceride transfer protein (MTTP)-I128T polymorphism, Nonalcoholic fatty liver disease (NAFLD) SNPs rs3772622, rs3772633, rs2276736, rs3772630 and rs3772627, and SNP rs3792323 in MAPKAPK3 associated with the outcome of IFN therapy in patients with HCV genotype 1b).

### 4. Evaluation of the in vitro differentiated cells with respect to their hepatocytic properties

Quantitative RT-PCR was used in order to perform a staging of the iPS-derived cells compared to different stages of liver development. An ABI-Taqman assay for albumin, AFP, HNF4a, CK18, Sox17, C/EPBa, and Mrp2 was used. These markers allow for a staging of the hepatic specification and therefore liver-precursor cells could be characterized in a development-stage specific manner. Other hepatic markers, such as albumin, AFP, alpha-1-antitrypsin, Mrp2 were determined by means of immuno-fluorescence and Western blot.

### 5. Establishing methods for the expansion of PCs from mouse and human

### 5.1 Conditional immortalization of human differentiated (endothelial) cells

Conditionally immortalized human endothelial cells derived from human umbilical-vein endothelial cells (HUVEC) were established. Sequential infections of cells were performed with a lentiviral vector for Dox-controlled expression of TAg (uni-TAg) and with leniviral vectors encoding hTert and c-myc. Two days after the second infection the cells were selected with G418. Resistant colonies appeared after two weeks. Cells were pooled and resulted in the expansion and immortalization of these cells.

Analysis of cell growth in the induced and uninduced states again revealed strict control of proliferation for both established cell lines. Strict dependence on the inducer was observed throughout the entire cultivation period. The differentiation status was confirmed by measuring the expression of endothelial specific markers and properties (PECAM-1 (CD31), CD34, uptake of acLDL, angiogenic capability) These phenotypic features were stable for at least 80 population doublings.

### 5.2 Evaluation and optimization of the infection of PCs

A prerequisite for an effective generation of cell lines is a high efficiency of the transduction of the immortalization cassettes. Thus, the primary PCs and iPSs were evaluated with respect to an efficient infection. For this, standard vectors were used that transduce GFP, and the conditions were optimized. Several strategies were evaluated, such as multiple infection cycles, alternative pseudotyping of the viruses (e.g. through MLV-env) and/or cell sorting strategies.

### 5.3 Development of vectors for the tet-mediated conditional expansion of liver progenitor cells

Using the strategy for a conditional immortalization (see 5.1) strategies for the expansion of liver cells were developed. Primarily, it was attempted to achieve an expansion of the hepatocytes through the conditional expression of suitable, and optionally, liver specific expansion genes, while maintaining their properties. For this, lentiviral vectors were constructed for the conditional expression of (liver) specific expansion genes, such as, for example, c-met, myc, hTert, and β-catenin. Furthermore, binary vectors for a liver specific activation of ubiquitary expansion cassettes are constructed. For this, the trans-activator is activated in a liver specific manner through defined promoters, and a tTA dependent activation of the expansion genes is achieved. Vectors are obtained that allow for a co-transduction of the binary expression cassette.

### 5.4 Development of expandable PCs

As a strategy for the expansion of liver progenitor cells (PC) while maintaining the differentiation potential into mature hepatocytes, liver progenitor cells were expanded in vitro through the time-controlled expression of genes that induce the expansion/proliferation of cells. This approach is based on the technology for a conditional immortalization of different cell types described above. Primarily, it was attempted to achieve an expansion of the hepatocytes through the conditional expression of suitable, and optionally, liver specific expansion genes, while maintaining their properties. After termination/silencing of these genes, the cells then develop into terminal hepatocytes. For the controlled expansion, the generated murine liver progenitor cells (PCs) of murine iPSs or mutated iPSs are infected with expansion vectors (as established) (see Figure 1). For this, several different expansion genes are used and evaluated. The cells as infected are cultivated and expanded in the presence of the inductor doxycycline (Dox). The expanded cultures were characterized regarding the regulation of the expansion genes, their proliferation in the presence and absence of Dox and the expression of relevant liver markers. Alternatively, the expansion vectors were introduced into the pluripotent cells (iPSs) and first differentiated in a "switched off" state into precursor cellsThe activation of the expansion cassettes then took place in a second step through the addition of dox in the progenitor cells. The cell cultures as produced were characterized with respect to the Dox-dependent expression of the expansion cassette, the expansion potential and the liver specific markers.

### 7. Evaluation of the expandable PCs

### 7.1 Standard evaluations

The expanded liver progenitor cells as generated were examined in regard to their pharmacometabolic properties by means of qRT-PCR and immuno fluorescence. In detail, the following phase I, II, and III-metabolization components were analyzed: Cytochrome p450, UDP-glucuronosyl transferase, bile salt transporter (BSEP), conjugate-export pump (MRP2).

## Claims

1. An *in vitro* method for producing induced stem cells (iSCs), such as induced pluripotent stem (iPS) cells, derived from a mammal which suffers from at least one adverse effect caused by a medical treatment of a disease or medical condition or the prevention thereof in said mammal,
wherein said mammal has a genetic defect or genetic predisposition that is linked with said adverse effect against said medical treatment of a disease or medical condition or the prevention thereof,
comprising reprogramming of somatic cells obtained from said mammal with at least one suitable reprogramming factor,
with the provisio that totipotent human stem cells are excluded.

2. The method according to claim 1, wherein said adverse effect against said medical treatment of a disease or medical condition or the prevention thereof, such as, for example, hepatotoxicity, transplant rejection, allergic reactions, weakness of the immune system, cancer, pain, fever, rash, sleep-wake-pattern, seizures, vertigo, nausea, delayed wound healing, blood clotting disorders, hyperactivity, and lack of bioavailability.

3. The method according to claim 1 or 2, wherein said genetic defect or genetic predisposition that is linked with said adverse effect is induced by external stimuli, such as, for example, said medical treatment of a disease or medical condition, or is present as inherent biological variation within a subpopulation of said mammal, such as a mutation, deletion, duplication, and/or SNP-related variation, in particular the polymorphisms CyP2C9; SNPs in N-actetyl transferases, such as Nat2; SNPs in thiopurine-S-methyl transferase (TPMT), microsomal triglyceride transfer protein (MTTP)-I128T polymorphism, SNPs rs3772622, rs3772633, rs2276736, rs3772630, rs3772627, and SNP rs3792323.

4. The method according to any of claims 1 to 3, wherein said reprogramming comprises
a) contacting one or more nuclear reprogramming factors or suitable conditions with said somatic cell, wherein said factor is preferably selected from a protein, a gene expressing said protein, a regulatory RNA, and a chemical substance, and more preferably selected from at least one gene product selected from an Oct family gene, a Klf family gene, and a gene product of a Sox family gene, preferably Oct4, Sox2, and Klf4, and, optionally, a Myc family gene, and preferably c-Myc, and optionally
b) the step of selecting and/or expanding the iSCs expressing said nuclear reprogramming factors, and preferably generating ES-like colonies.

5. The method according to any of claims 1 to 4, further comprising the step of identifying said iSCs, and preferably said iPSs, through analytical methods such as teratome formation experiments, qRT-PCR for markers of pluripotency, and/or chimerism analyses following blastocyst-injection.

6. The method according to any of claims 1 to 5, further comprising the step of a reversion of the expression of the factors as expressed, preferably through conditional silencing of the expression, excision of said factors, for example through a cre/lox system, and/or genetic silencing of said factors.

7. The method according to any of claims 1 to 6, further comprising the step of differentiating said iSCs, and preferably said iPSs, into cells of a desired phenotype, preferably neuronal cells, cardiomyocytes, hepatocytes, and bone marrow cells, comprising the use of instructive cytokines, such as, for example, activin A, BMP4, bFGF, VEGF, Wnt, and/or Notch, wherein said cells preferably are transduced with a selection construct, harboring a phenotype-specific, preferably a neuronal cell-, cardiomyocyte-, hepatocyte-, or bone marrow cell-specific promoter controlling marker proteins, such as GFP, GFP-variants or antibiotic-resistance genes, such as neomycin and/or puromycin.

8. The method according to claim 7, wherein said method further comprises subjecting said cells of a desired phenotype to one or more factors or conditions controlling the proliferation of said cells, such as a) defined culture conditions, b) cytokines, such as bFGF, c) expression of recombinant expression of genes such as c-myc, and/or SCF, TERT, SV40 Large T antigen, HPV16 E6, HPV16 E7, Bmil, Fbx15, ERas, ECAT15-2, Tcl1, catenin, ECAT1, Esg1, Dnmt3L, EAT8, Gdf3, Sox15, ECAT15-1, Fthl17, Sall4, Rex1, UTF1, Stella, Stat3, and Grb2, d) expression of regulatory RNAs, and/or e) application of proliferation modifying/controlling chemical substances.

9. The method according to any of claims 1 to 8, wherein said factors are recombinantly introduced into said somatic cell through a genetic expression construct, such as through a viral expression construct, wherein said genetic expression can be preferably regulated by adding external factors, such as doxycycline and/or genetic recombination methods, such as through a cre/lox system.

10. An iSC-cell line, preferably an iPS-cell line, produced according to any of claims 1 to 6, or a transgenic mammalian cell line, produced according to any of claims 7 to 9, or a transgenic non-human mammal, comprising said transgenic cell line.

11. An *in vitro* method for diagnosing for at least one adverse effect caused by a medical treatment of a disease or medical condition or the prevention thereof in a mammal, comprising the steps of:
a) providing an iSC-cell line, preferably an iPS-cell line, or a transgenic mammalian cell line, or a transgenic non-human mammal according to claim 10,
b) contacting said cell line or animal with a candidate compound, either alone or in combination with at least one additional compound,
c) determining if the cells undergo any change in morphology, marker expression, or functional activity being attributable to said compound, compared to a cell line derived from a mammal lacking said genetic defect or genetic predisposition that is linked with said adverse effect against said medical treatment of a disease or medical condition or the prevention thereof, and
d) concluding on said at least one adverse effect caused by said medical treatment of a disease or medical condition or the prevention thereof in said mammal.

12. An *in vitro* method for screening for a compound for a medical treatment of a disease or medical condition or the prevention thereof in a mammal lacking or having reduced side-effects, comprising the method according to claim 11, wherein no change in morphology, marker expression, or functional activity being attributable to said compound is indicative for a treatment lacking or having reduced side-effects.

13. An *in vitro* method for screening for a compound reverting or reducing at least one adverse effect caused by a medical treatment of a disease or medical condition or the prevention thereof in a mammal, comprising the method according to claim 19 in the presence of at least one additional compound in step b), wherein no change in morphology, marker expression, or functional activity being attributable to said additional compound in step b) is indicative for a compound reverting or reducing at least one adverse effect caused by said medical treatment of a disease or medical condition or the prevention thereof.

14. A method for producing a pharmaceutical composition, comprising a method according to claim 12 or 13, and formulating said compound lacking or having reduced side-effects and/or said compound reverting or reducing at least one adverse effect caused by said medical treatment of a disease or medical condition or the prevention thereof together with a pharmaceutically acceptable carrier.

15. Use of a pharmaceutical composition produced according to claim 14 for the treatment of an adverse effect against a medical treatment of a disease or medical condition or the prevention thereof, wherein said adverse effect is preferably selected from hepatotoxicity, neurotoxicity, transplant rejection, allergic reactions, weakness of the immune system, cancer, pain, fever, rash, sleep-wake-pattern, seizures, vertigo, nausea, delayed wound healing, blood clotting disorders, hyperactivity, and lack of bioavailability, preferably for the treatment of a disease selected from liver diseases, proliferative disorders, such as cancer, inflammatory diseases, graft-versus-host reactions, viral or bacterial infections, trauma, depression, diabetes, obesity, allergic reactions, and stroke, wherein said treatment lacks or has reduced side-effects.
